# EUROPEAN PATENT APPLICATION

(11) **EP 2 353 510 A2**
(43) Date of publication of application: **10.08.2011**
(21) Application number: 11250114.3
(22) Date of filing: 02.02.2011
(51) Int. Cl.: A61B 17/00

(54) **Surgical retrieval apparatus**

(30) Priority: 03.02.2010 US 301126 P; 22.12.2010 US 975756
(71) Applicant: Tyco Healthcare Group LP, New Haven, CT 06511 (US)
(72) Inventor: Collier, Nicholas J., Burwell, Cambridge CB25 9JG (GB); Fleming, Alistair I., Lower Cambourne, Cambridgeshire CB23 6ER (GB); Scott, Natalie, Cambridge, CB1 3AW (GB)
(74) Representative: Soames, Candida Jane

(57) **Abstract**

A surgical retrieval apparatus (100) includes an elongate flexible tubular member (110) having a distal opening and a lumen and a support member (140) having a chamber formed therein in fluid communication with the tubular member and movable from a first position to a second expanded position in response to introduction of fluid into the chamber. A retrieval bag (130) extends from the support member and has a first end (132) and a closed second end (134). The first end of the retrieval bag is movable to an open configuration when the expandable member transitions from the first position to the second expanded position.

## Description

### BACKGROUND

This application claims priority from provisional application serial no. 61/301,126, filed February 3, 2010, the entire contents of which are incorporated herein by reference.

### 1. Technical Field

The present disclosure relates to a surgical containment apparatus. More particularly, the present disclosure relates to a specimen retrieval apparatus for use in minimally invasive surgical procedures.

### 2. Background of Related Art

In minimally invasive surgical procedures operations are carried out within the body by using elongated instruments inserted through small entrance openings in the body. The initial opening in the body tissue to allow passage of instruments to the interior of the body may be a natural passageway of the body, or it can be created by a tissue piercing instrument such as a trocar, or created by a small incision into which a cannula is inserted.

Because the tubes, instrumentation, and any required punctures or incisions are relatively small, the surgery is less invasive as compared to conventional surgical procedures in which the surgeon is required to cut open large areas of body tissue. Therefore, minimally invasive surgery minimizes trauma to the patient and reduces patient recovery time and hospital costs.

Minimally invasive procedures may be used for partial or total removal of body tissue or organs from the interior of the body, e.g. nephrectomy, cholecystectomy, lobectomy and other procedures including thoracic, laparoscopic and endoscopic procedures. During such procedures, it is common that a cyst, tumor, or other affected tissue or organ needs to be removed via the access opening in the skin, or through a cannula. Various types of entrapment devices have been disclosed to facilitate this procedure. In many procedures where cancerous tumors are removed, removal of the specimen in an enclosed environment is highly desirable to prevent seeding of cancer cells.

In minimally invasive thoracic surgery, access to the thoracic cavity is limited as well as maneuverability within the cavity as the access port is placed between the confined space between a patient's ribs. Such procedures, commonly referred to as video assisted thorascopic surgery (VATS), aim to reduce patient recovery time by accessing the thoracic cavity through the natural intercostal space without spreading the ribs as in open procedures. This restricted access can sometimes cause problems when removing large specimens. Moreover, in such procedures, e.g. thorascopic wedge resection and lobectomy, it is often necessary to remove a portion of the lung and retrieve it relatively intact for pathology. It is also important that the specimen be sufficiently contained to prevent seeding of cancer cells during manipulation and removal.

In designing such specimen retrieval instrumentation, a balance must be struck between the need to provide a retrieval apparatus with a strong enough containment bag to prevent tearing or rupture while providing sufficient rigidity to enable manipulation and removal. Another balance which needs to be achieved is to provide sufficient maneuverability while reducing tissue trauma, e.g. damaging lung tissue, during manipulation and removal. Additionally, the instrumentation on one hand should be able to be inserted through a small access incision or port while on the other hand able to accommodate a wide range of patient sizes and be able to easily remove large specimens and minimize risk of seeding.

It would therefore be advantageous to provide a specimen retrieval device for minimally invasive surgical procedures with increased maneuverability and which minimizes trauma to surrounding tissue and which successfully achieves the balance of competing factors enumerated above.

### SUMMARY

The present disclosure is directed to a surgical retrieval apparatus. The present disclosure provides in one aspect a surgical retrieval apparatus comprising an elongate flexible tubular member having a distal opening and a lumen, a support member having a chamber formed therein in fluid communication with the tubular member and movable between a first collapsed insertion position to a second expanded position in response to introduction of fluid into the chamber, and a retrieval bag extending from the support member and having a first end and a closed second end. The first end of the retrieval bag is movable to an open configuration when the expandable member transitions from the first position to the second expanded position.

Preferably, the support member transitions from the second position to the first position upon removal of the fluid from the chamber of the support member.

In some embodiments, the retrieval bag includes a port spaced from the support member, the port adapted to receive a suction device to remove air from the retrieval bag. In some embodiments, the chamber of the support member receives air from an air pump. In other embodiments, the chamber receives an expandable foam.

In some embodiments, the retrieval bag has a plurality of ribs extending from an inside surface. In other embodiments, the retrieval bag has a textured surface on an inside surface. In other embodiments, the retrieval bag has a plurality of air channels extending lengthwise thereof.

In another aspect, the present disclosure provides a surgical retrieval apparatus comprising an elongated member, a support member adjacent a distal portion of the elongated member, and a specimen retrieval bag supported by the support member. The retrieval bag includes internal structure to prevent a tissue specimen contained therein from slipping to the bottom of the retrieval bag and balling at the bottom of the retrieval bag during removal of the retrieval bag from a patient. The internal structure of the retrieval bag can include a textured surface on an inside surface and/or a plurality of struts.

The present disclosure also provides in another aspect a surgical retrieval apparatus comprising an elongated member, a support member adjacent a distal portion of the elongated member, and a specimen retrieval bag supported by the support member. The retrieval bag includes an opening and an air port spaced from the support member for receipt of a suction device to collapse the bag upon application of a vacuum. The retrieval bag in some embodiments has a textured surface on an inside surface. In some embodiments, the support member is inflatable.

In another aspect, the present disclosure provides a method of retrieving a tissue sample comprising:
a) inserting a surgical retrieval apparatus through an opening in a patient's skin, the surgical retrieval apparatus including:
   an elongate tubular member,
   a support member having a chamber therein to receive fluid; and
   a retrieval bag extending from the support member and having a first end and a closed second end, the first end movable between an open configuration and a closed configuration;
b) introducing a fluid into the chamber of the support member to move the support member from a first position to a second expanded position to move the first end of the retrieval bag into the open configuration;
c) positioning the retrieval bag in proximity to the tissue sample;
d) moving the tissue sample into the retrieval bag through the first end of the retrieval bag;
e) applying a vacuum to the interior of the retrieval bag while the tissue sample is contained therein to remove air from the retrieval bag and collapse the retrieval bag around the specimen; and
f) removing the retrieval bag from the patient's body.

In some embodiments, the retrieval bag has a port on an exterior thereof and the step of applying a vacuum includes positioning a suction device in communication with the port. In some embodiments, the step of introducing a fluid comprises introducing air into the chamber by application of an air pump. In other embodiments, the step of introducing a fluid comprises introducing an expandable foam.

In some embodiments, the step of inserting the surgical apparatus includes the step of inserting the apparatus through an access port into the thoracic cavity.

### BRIEF DESCRIPTION OF THE DRAWINGS

Embodiments of the presently disclosed specimen retrieval apparatus are described hereinbelow with reference to the drawings wherein:
FIG. 1 is a perspective view of the retrieval bag of the specimen retrieval apparatus of the present disclosure in the collapsed insertion position;
FIG. 2 is perspective view of the specimen retrieval apparatus of the present disclosure showing the retrieval bag of Figure 1 in the expanded position;
FIG. 3 is a cross-sectional view taken along line 3-3 of Figure 2;
FIG. 4 is a perspective view of an alternate embodiment of the specimen retrieval bag of the present disclosure having an interior textured surface;
FIG. 5 is a cross-sectional view taken along line 5-5 of Figure 4;
FIG. 6 is a perspective view of another alternate embodiment of the specimen retrieval bag having a plurality of struts (ribs);
FIG. 7 is a cross-sectional view taken along line 7-7 of Figure 6;
FIG. 8 is a perspective view of yet another alternate embodiment of the specimen retrieval bag of the present disclosure having a plurality or air channels;
FIG. 9 is a cross-sectional view taken along line 9-9 of Figure 8;
FIGS. 10-14 illustrate the steps of withdrawing a specimen utilizing the specimen retrieval bag of FIG. 1, wherein:

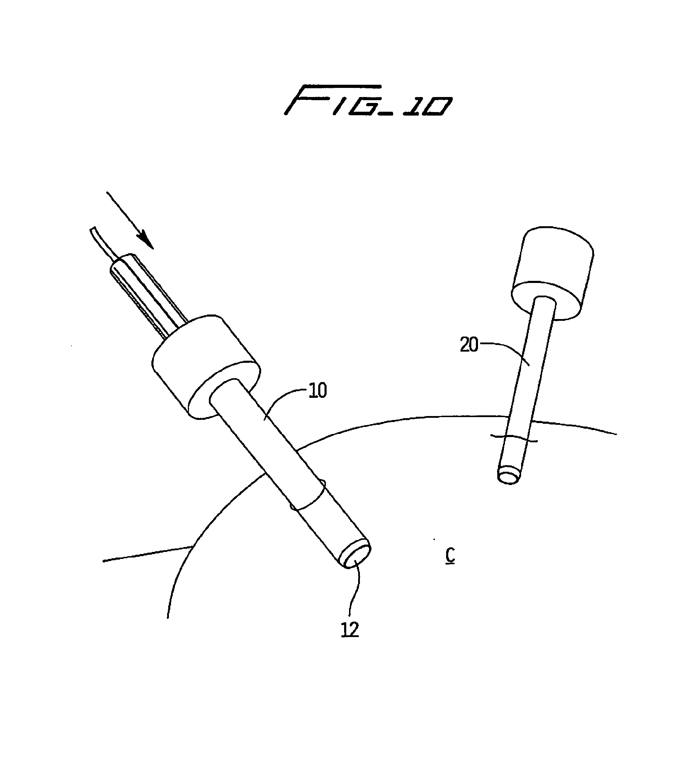

   FIG. 10 illustrates two access ports inserted through the patient's body for access to the body cavity (the body and cavity shown schematically);

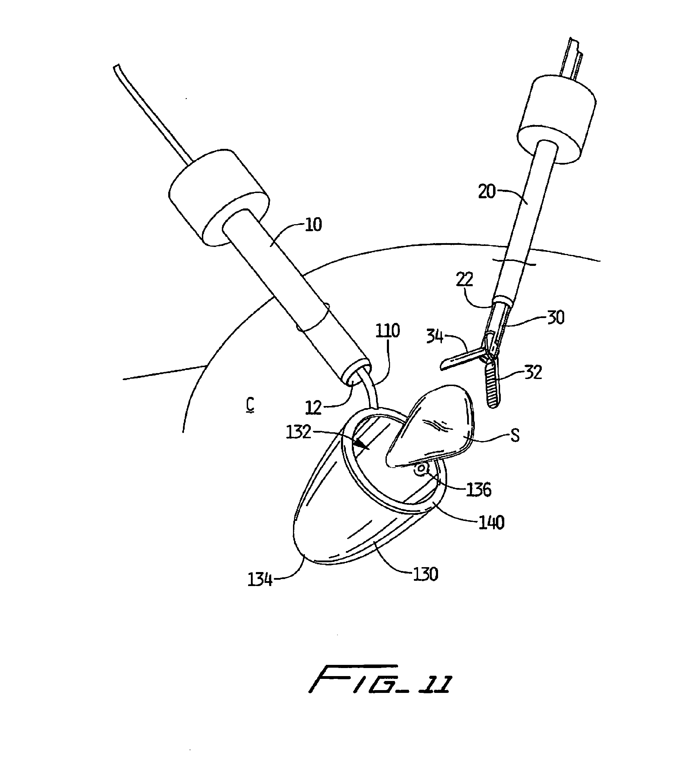

   FIG. 11 illustrates the retrieval bag in the open position and a grasper placing the specimen in the retrieval bag;

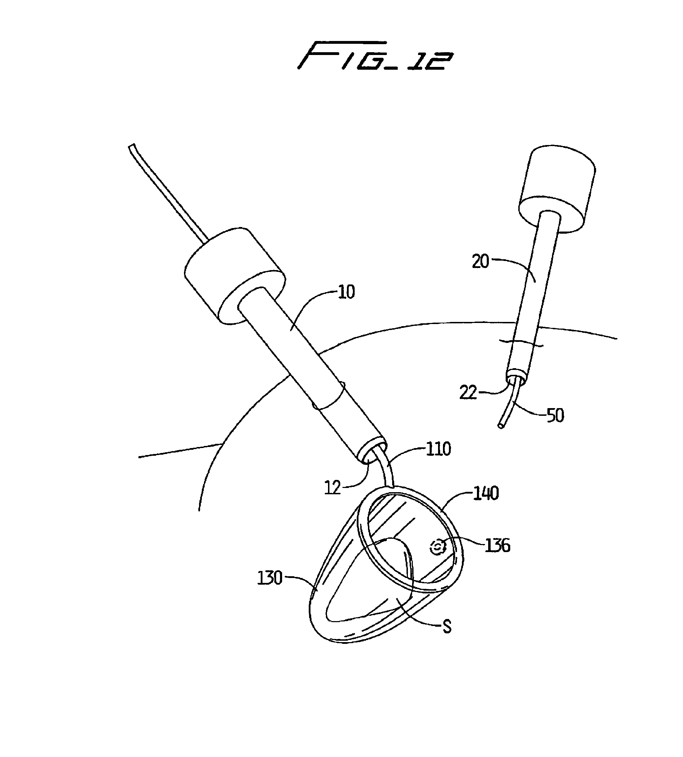

   FIG. 12 illustrates the specimen positioned in the retrieval bag and a suction device being inserted through the grasper port after the grasper has been removed from the body cavity;

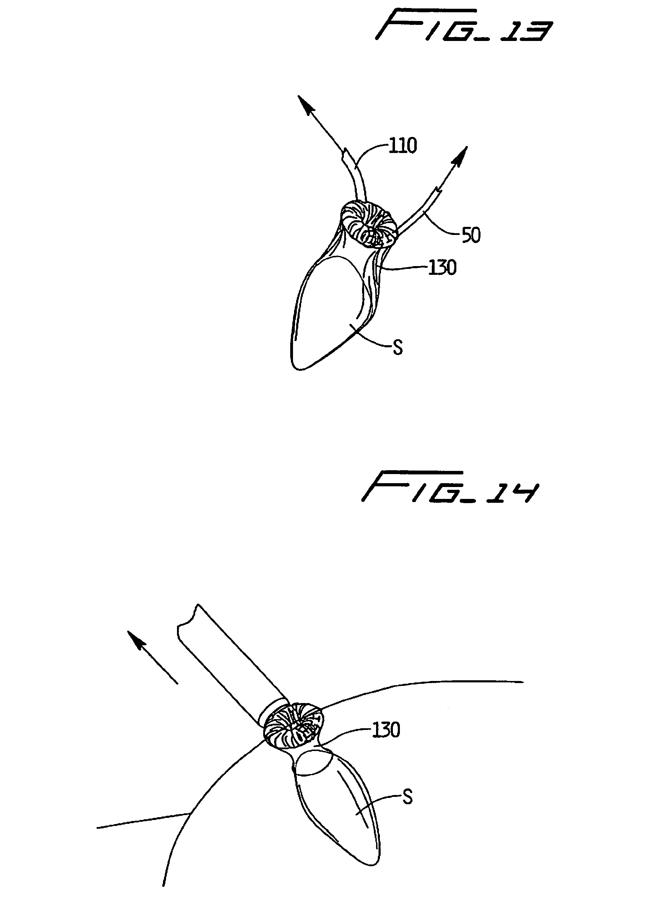

   FIG. 13 illustrates the vacuum applied to the interior of the retrieval bag to collapse the bag around the specimen; and
   FIG. 14 illustrates the retrieval bag being withdrawn from the patient's cavity.

### DETAILED DESCRIPTION OF THE EMBODIMENTS

Embodiments of the present disclosure will now be described in detail with reference to the drawings, in which like reference numerals designate identical or corresponding elements in each of the several views. As used herein, the term distal refers to the portion of the instrument which is further from the user while the term proximal refers to that portion of the instrument which is closer to the user.

The surgical retrieval apparatus disclosed herein may find use in any procedure where access to the interior of the body is limited to a relatively small incision, with or without the use of a cannula, as in minimally invasive procedures. The devices herein may find particular use in minimally invasive thoracic surgery where access to the thoracic cavity is through a space located between adjacent ribs known as the intercostal space.

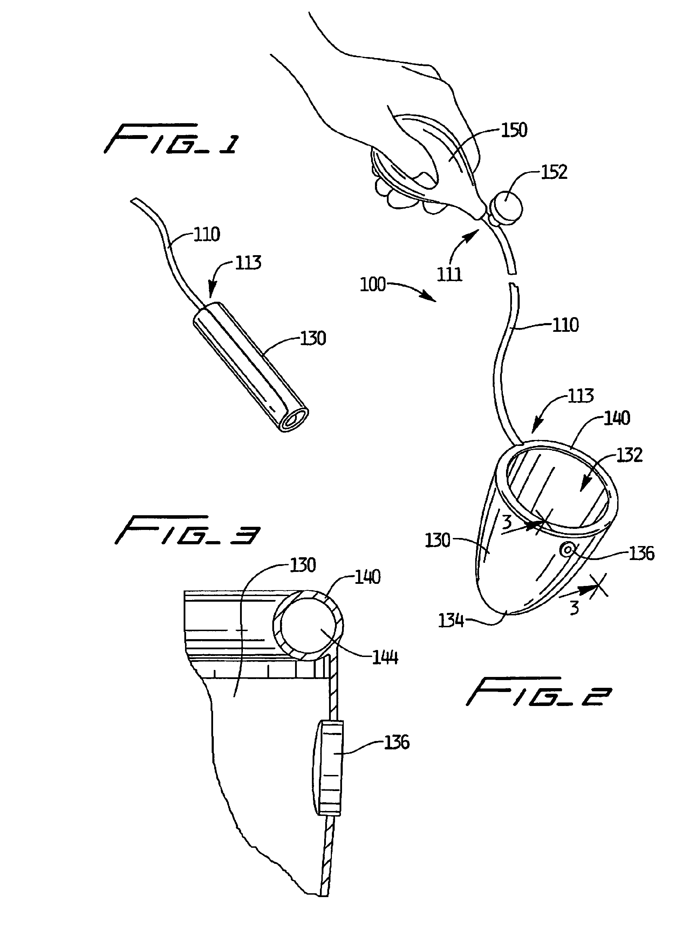

Referring initially to FIGS. 1 and 2, a surgical retrieval apparatus 100 is illustrated. Surgical retrieval apparatus 100 is preferably configured and dimensioned for use in minimally invasive surgical procedures (e.g. thoracic, laparoscopic, endoscopic, procedures). Surgical retrieval apparatus 100 includes an elongated flexible tubular member 110 and a retrieval bag 130. The retrieval bag 130 is supported by a support member 140 in the form of a circumferential ring or rim. The ring 140 is formed in a tubular configuration in that it contains a chamber or channel 144. In this manner, the ring 140 can be inflated to expand/open the retrieval bag 130.

A hand pump 150 is shown in Figure 2, with a valve 152, for inflating the ring 140. Alternatively a foot pump (not shown) can be provided. The pump 150 is attached to a proximal end 111 of tubular member 110. Distal end 113 of tubular member 110 is attached to ring 140 to provide for fluid communication with the internal chamber 144 of ring 140.

A second air port 136 is positioned on the retrieval bag 130 as will be described in more detail below in conjunction with the method of use and Figures 10-14.

In use, the retrieval bag 130 can be delivered in a collapsed (e.g. rolled) configuration through an access port such as in the configuration shown in Figure 1. As can be appreciated, by delivering the bag 130 in the collapsed configuration without an external sleeve thereover, the overall profile of the apparatus is minimized which enables a smaller diameter access port to be utilized and/or facilitate movement within the limited space of the body cavity, e.g. thoracic cavity. Once inserted inside the cavity, pump 150 external to the body is actuated to inject air into the circumferential chamber 144 in ring 140 to expand the ring 140 circumferentially to the expanded position of Figure 2. Expansion of the ring 140 expands the mouth or opening 132 of bag 130 due to the attachment of the bag to the ring 140. As shown in Figure 2, the retrieval bag 130 has an open end 132 at its mouth and a closed end 134 at the opposite end. Inflation of the ring 140 can be controlled by the amount of air pumped into the channel 144. In this manner, the degree of expansion of the rim 140 and the extent of opening the mouth 132 of the bag 130 can be controlled to accommodate smaller spaces within the cavity.

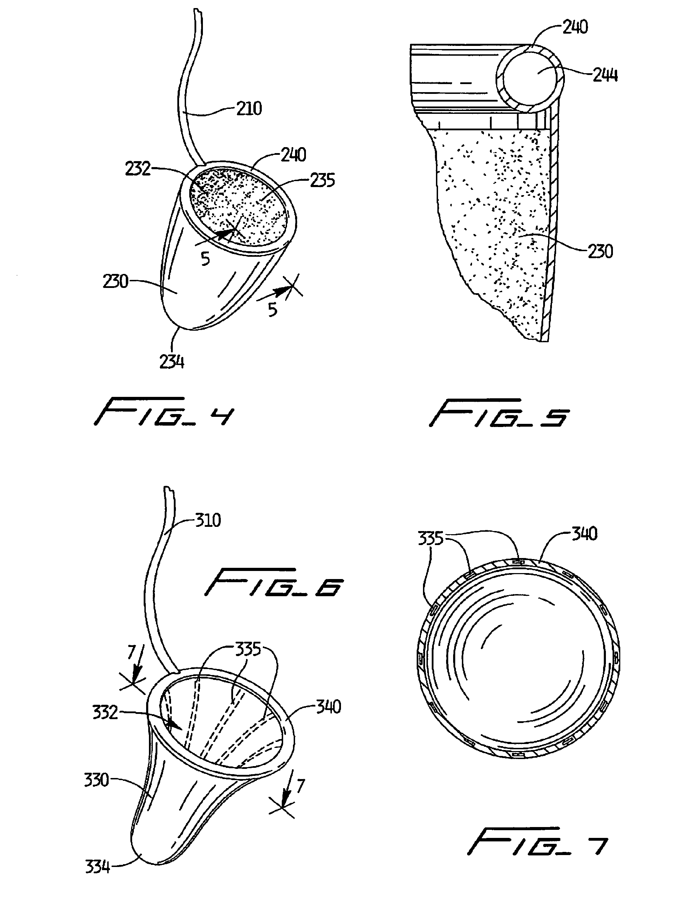

In an alternate embodiment of Figures 4 and 5, the retrieval bag 230 is similar to retrieval bag 130 in all respects except for the textured surface 235 on the interior of the bag and the absence of a second air port. It should however be appreciated that a second air port can be provided to work in conjunction with the textured surface. The textured surface 235 functions to limit the slippage of the captured specimen to the bottom, e.g. toward closed end 234, of the bag 230 and prevent the balling of such specimen to facilitate removal through the incision. The texture can extend from the mouth 232 of the retrieval bag 230 to the closed end 234. Alternatively, it can be provided only on certain regions of the bag 230. The support member or ring 240 of bag 230, like rim 140 of Figure 1, has a fluid chamber 244 communicating with flexible tubular member 210 for expansion of the ring (rim) 240 to open bag 230 by receipt of fluid.

In an alternate embodiment of Figures 6 and 7, instead of a textured surface as in the bag 230 of Figure 4, the retrieval bag 330 extending from support member 340 has a series of elongated struts or splines 335 circumferentially arranged on the interior surface thereof. The struts 335 can extend along the length of the bag 330 from the mouth 332 adjacent ring (rim) 340 to the closed end 334 of the bag 330. Although struts (ribs) 335 are shown extending the length of the bag 330 and substantially equidistantly spaced around the perimeter of the bag 330, it should be appreciated that any number of struts can be provided and in various configurations and various positions within the bag 330 to achieve its function. The struts function to limit the slippage of the captured specimen to the bottom of the bag 330 and limit the balling of such specimen to facilitate removal. As in the embodiment of Figures 1-3, the bag 330 is attached to ring 340 which has a fluid chamber therein in fluid communication with flexible tubular member 310.

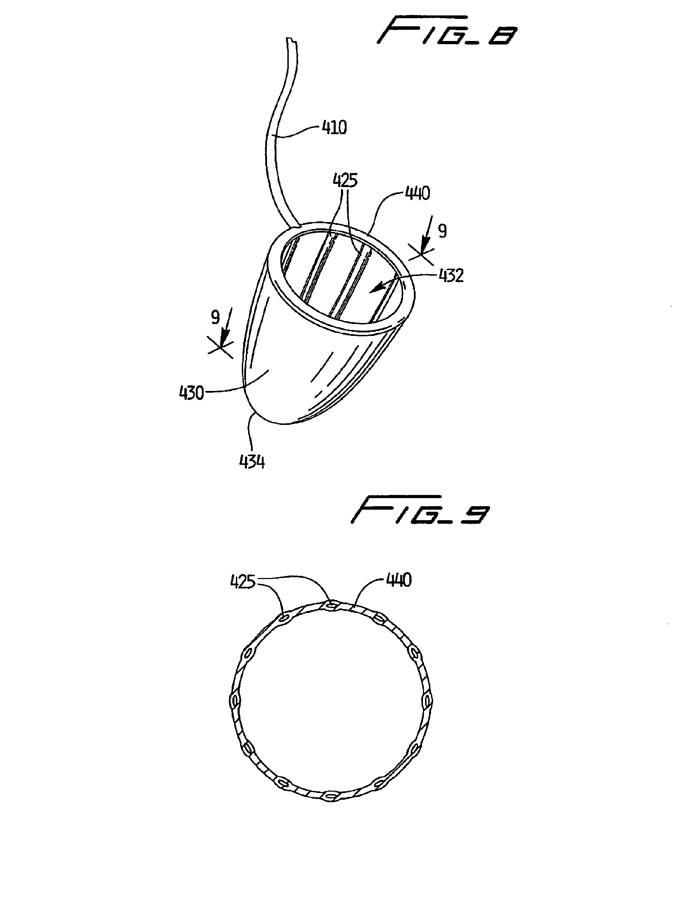

In the embodiment of Figures 8 and 9, a series of air channels 425 are formed along the length of the bag 430. These air channels 425 are in fluid communication with support member (ring) 440 which is in fluid communication with flexible tubular member 410. When air or fluid is introduced into the support member 440 to expand the support member 440 to open the bag 430, the air channels 425 are also expanded, thereby assisting the unfurling of the bag 430. The air channels 425 are shown extending along the length of the bag, from the mouth 432 to the closed end 434, and substantially equidistantly spaced. A different number of air channels 425 can be provided and in various arrangements. A textured surface and/or struts can optionally be provided to further prevent slippage of the specimen. A second air port can optionally be provided to collapse the bag 430 as described with respect to bag 130 of the Figure 1 embodiment.

Turning now to Figures 10-14, use of the specimen retrieval apparatus will now be described, with use of retrieval apparatus 100 of Figure 1 shown and described by way of example.

As shown in Figure 10, a first access port 10 and a second access port 20 extend through the skin of the patient and into the body cavity C, such as a thoracic cavity. The patient's body and cavity are shown schematically, it being understood that the surgical retrieval apparatus of the present disclosure can be used in the thoracic cavity, the abdominal cavity and other areas of the body for minimally invasive surgery. The specimen retrieval apparatus 100 is placed through access port or cannula 10 in the collapsed position of Figure 1 and advanced through port opening 12 of port 10. Once inserted and maneuvered to a desired position in the cavity, the pump, e.g. pump 150 of Figure 2, is actuated to introduce air through tubular member 110 into the support member (rim) 140 to inflate the support member 140 and open the mouth of the bag 130. The degree of inflation can be controlled by the amount of air forced into the chamber of the support member 140.

A grasper 30 is inserted through access port or cannula 20 and advanced through distal opening 22 as shown in Figure 11. The grasper 30 grasps the specimen between its jaws 32, 34 and places the specimen S through the mouth of the open retrieval bag 130 and into the bag as shown in Figure 11. The grasper 30 can also be utilized to maneuver the bag 130 over the specimen S. The specimen is now ready for removal. The grasper 30 is then removed and a suction device 50 is inserted through port 20, exiting distal opening 22 (see Figure 12). Alternatively, suction device 50 can be inserted through another port.

The support member 140 is deflated by applying a vacuum through tubular member 110, causing it to contract and close the mouth 132 of bag 130, trapping the specimen inside and sealing off the bag 130. The bag 130 can further be clipped to further close it off.

The suction device 50 is inserted through the second air port 136 in retrieval bag 130, and can be connected thereto. Activation of the vacuum removes the air from the bag 130 surrounding the specimen S, thereby collapsing the bag 130 around the specimen S. This suction reduces the size of the bag 130 (see Figures 13 and 14), in a "vacuum packaging" manner, to facilitate removal through the port 10 or if a port is not being used, removal directly through the access incision. Application of the suction by suction device 50 also collapses the bag 130 to prevent the specimen S from slipping or balling at the bottom of the bag 130. This also facilitates removal as the specimen maintains its shape and position within the bag 130. That is, the specimen can be maintained such that its long axis is substantially perpendicular to the incision which reduces the force required for removal through the incision or port. Also, as can be appreciated, the orientation and shape of the specimen S is substantially maintained to facilitate not only removal but pathology. Moreover, any compression or stretching of the specimen prior to removal occurs inside the bag which minimizes the risk of seeding.

The surgeon can pull the flexible tube to pull the bag through the port 10. Alternatively, to remove the apparatus 100, the surgeon can grasp the proximal end of the bag 130 or the support member 140 and pull it through the port 10. If a port is not utilized, the surgeon can lift the bag 130 to the incision, clip the neck closed and grasp the proximal end of the bag and pull it directly through the incision.

In the embodiment of Figure 6 with the elongated air channels 335, the air from the air channel 335 is removed along with the air from the support member 340 to collapse the bag 330 for removal.

It should be noted that the retrieval bags of the other embodiments can be utilized in a similar fashion as retrieval bag 130 of Figures 10-14, except that the bags themselves have internal structure as described above to help prevent the specimen from slipping to the bottom of the bag. Thus, these retrieval apparatus can be utilized without the addition suction device 50. However, to further facilitate removal, the bags of these embodiments can optionally be provided with an air port to receive suction device 50 to collapse the bag as in Figure 13 and 14.

Markings can be provided along the length of the bag to indicate how near the bottom the sample is.

A lubricious coating can be placed on the external surface of the specimen retrieval bags described herein to facilitate removal through the port or incision. A lubricious coating can also be placed on an internal surface of the port, also to facilitate removal.

Various other sources of fluid for expanding the support members include pressurized gases (e.g. carbon dioxide) or liquids (e.g. saline). Other biocompatible fluids may be used as well. Suitable biocompatible foams known in the art can also be used for introduction into the chamber to cause expansion of the support member. A foam material can in some instances provide a more rigid support member than using a gas.

The expandable ring support member can reduce trauma to surrounding tissue. The inflatable ring allows the practitioner to control the amount of inflation. This allows the practitioner increased flexibility when performing surgical procedures.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:-
1. A surgical retrieval apparatus comprising: an elongate flexible tubular member having a distal opening and a lumen; a support member in fluid communication with the tubular member, the support member having a chamber formed therein and movable between a first collapsed insertion position and a second expanded position in response to introduction of a fluid to the chamber; and a retrieval bag extending from the support member, the retrieval bag having a first end and a closed second end, the first end transitionable between a closed and an open configuration when the expandable member transitions from the first position to the second position.
2. The surgical retrieval apparatus of paragraph 1, wherein the expandable member transitions from the second position to the first position upon removal of the fluid from the chamber of the support member.
3. The surgical retrieval apparatus of paragraph 1, wherein the retrieval bag includes a port spaced from the support member, the port adapted to receive a suction device to remove air from the retrieval bag.
4. The surgical retrieval apparatus of paragraph 1, wherein the chamber of the support member receives an expandable foam.
5. The surgical retrieval apparatus of paragraph 1, wherein the retrieval bag has a plurality of ribs on an inside surface.
6. The surgical retrieval apparatus of paragraph 1, wherein the retrieval bag has a textured surface on an inside surface.
7. The surgical retrieval apparatus of paragraph 1, wherein the retrieval bag has a plurality of air channels extending lengthwise thereof, the air channels expandable upon expansion of the support member.
8. The surgical retrieval apparatus of paragraph 5, wherein the retrieval bag has a textured surface on an inside surface.
9. The surgical retrieval apparatus of paragraph 6, wherein the retrieval bag has a plurality of air channels extending lengthwise thereof.
10. A surgical retrieval apparatus comprising an elongated member, a support member adjacent a distal portion of the elongated member, and a specimen retrieval bag supported by the support member, the retrieval bag includes internal structure to prevent a tissue specimen contained therein from slipping to the bottom of the retrieval bag and balling at the bottom of the retrieval bag during removal of the retrieval bag from a patient.
11. The surgical retrieval apparatus of paragraph 10, wherein the internal structure of the retrieval bag includes a textured surface on an inside surface.
12. The surgical retrieval apparatus of paragraph 10, wherein internal structure includes a plurality of struts.
13. A surgical retrieval apparatus comprising an elongated member, a support member adjacent a distal portion of the elongated member, and a specimen retrieval bag supported by the support member, the retrieval bag including an opening and an air port spaced from the opening for receipt of a suction device to collapse the bag upon application of a vacuum.
14. The surgical retrieval apparatus of paragraph 13, wherein the retrieval bag has a textured surface on an inside surface.
15. The surgical retrieval apparatus of paragraph 13, wherein the support member is inflatable by a tube connected to the support member.
16. A method of retrieving a tissue sample comprising: a) inserting a surgical retrieval apparatus through an opening in a patient's skin, the surgical retrieval apparatus including: an elongate tubular member, a support member having a chamber therein to receive fluid; and a retrieval bag extending from the support member and having a first end and a closed second end, the first end movable between an open configuration and a closed configuration; b) introducing a fluid into the chamber of the support member to move the support member from a first position to a second expanded position to move the first end of the retrieval bag into the open configuration; c) positioning the retrieval bag in proximity to the tissue sample; d) moving the tissue sample into the retrieval bag through the first end of the retrieval bag; e) applying a vacuum to the interior of the retrieval bag while the specimen is retained therein to remove air from the retrieval bag and collapse the retrieval bag around the specimen; and f) removing the retrieval bag from the patient's body.
17. The method of paragraph 16, wherein the retrieval bag has a port and the step of applying a vacuum includes positioning a suction device in communication with the port.
18. The method of paragraph 16, wherein the step of introducing a fluid comprises introducing one of air or foam into the chamber.
19. The method of paragraph 16, wherein the retrieval bag includes a plurality of elongated air channels and the step of introducing a fluid into the chamber expands the air channels.
20. The method of paragraph 14, wherein the step of inserting the surgical apparatus includes the step of inserting the apparatus through an access port into the thoracic cavity.

## Claims

1. A surgical retrieval apparatus comprising an elongated member, a support member adjacent a distal portion of the elongated member, and a specimen retrieval bag supported by the support member, the retrieval bag including an opening and an air port spaced from the opening for receipt of a suction device to collapse the bag upon application of a vacuum.

2. The surgical retrieval apparatus of claim 1, wherein the retrieval bag has a textured surface on an inside surface.

3. The surgical retrieval apparatus of claims 1 or 2, wherein the support member is inflatable by a tube connected to the support member.

4. The surgical retrieval apparatus of any of claims 1-3, wherein the elongated member is a flexible tubular member having a distal opening and a lumen and the support member is in fluid communication with the tubular member, the support member having a chamber formed therein and movable between a first collapsed insertion position and a second expanded position in response to introduction of a fluid to the chamber.

5. The surgical retrieval apparatus of any of claims 1-4, wherein the retrieval bag has a first end and a closed second end, the first end transitionable between a closed and an open configuration when the support member transitions from the first position to the second position.

6. The surgical retrieval apparatus of claims 4 or 5, wherein the expandable member transitions from the second position to the first position upon removal of the fluid from the chamber of the support member.

7. The surgical retrieval apparatus of any of claims 4-6, wherein the chamber of the support member receives an expandable foam.

8. The surgical retrieval apparatus of any of claims 1-7, wherein the retrieval bag has a plurality of ribs on an inside surface.

9. The surgical retrieval apparatus of any of claims 1-8, wherein the retrieval bag has a plurality of air channels extending lengthwise thereof, the air channels expandable upon expansion of the support member.

10. A surgical retrieval apparatus comprising an elongated member, a support member adjacent a distal portion of the elongated member, and a specimen retrieval bag supported by the support member, the retrieval bag includes internal structure to prevent a tissue specimen contained therein from slipping to the bottom of the retrieval bag and balling at the bottom of the retrieval bag during removal of the retrieval bag from a patient.

11. The surgical retrieval apparatus of claim 10, wherein the internal structure of the retrieval bag includes a textured surface on an inside surface.

12. The surgical retrieval apparatus of claims 10 or 11, wherein the internal structure includes a plurality of struts.

13. The surgical retrieval apparatus of any of claims 10-12, wherein the elongated member is a flexible tubular member having a distal opening and a lumen and the support member is in fluid communication with the tubular member, the support member having a chamber formed therein and movable between a first collapsed insertion position and a second expanded position in response to introduction of a fluid to the chamber.

14. The surgical retrieval apparatus of any of claims 1-13, wherein the retrieval bag has a first end and a closed second end, the first end transitionable between a closed and an open configuration when the support member transitions from the first position to the second position.

15. The surgical retrieval apparatus of claims 13 or 14, wherein the expandable member transitions from the second position to the first position upon removal of the fluid from the chamber of the support member.
